(19) **Europäisches Patentamt**

**European Patent Office**

**Office européen des brevets**

(11) Publication number: **0 266 216 B1**

(12) **EUROPEAN PATENT SPECIFICATION**

(45) Date of publication of patent specification: **15.12.93**

(51) Int. Cl.⁵: **G01N 33/96**, //G01N33/52, G01N33/66

(21) Application number: **87309618.4**

(22) Date of filing: **30.10.87**

(54) **Control or calibration solutions for fluid analysis.**

(30) Priority: **31.10.86 JP 259797/86**
**18.11.86 JP 274919/86**

(43) Date of publication of application:
**04.05.88 Bulletin 88/18**

(45) Publication of the grant of the patent:
**15.12.93 Bulletin 93/50**

(84) Designated Contracting States:
**DE GB**

(56) References cited:
**FR-A- 2 139 703**
**US-A- 3 016 292**
**US-A- 3 977 995**

(73) Proprietor: **FUJI PHOTO FILM CO., LTD.**
**210 Nakanuma**
**Minami Ashigara-shi**
**Kanagawa 250-01(JP)**

(72) Inventor: **Terashima, Masaaki, c/o Fuji Photo**
**Film Co.Ltd.**
**No. 11-46, Senzui 3-chome**
**Asaka-shi, Saitama(JP)**

(74) Representative: **Arthur, Bryan Edward et al**
**Withers & Rogers**
**4 Dyer's Buildings**
**Holborn**
**London EC1N 2JT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

**Description**

FIELD OF THE INVENTION

The present invention relates to control or calibration solutions used for daily control of the precision of analysis and for making a calibration curve in analyzing quantitatively a target component (an analyte) in samples of organic body fluids, such as whole blood, using dry analysis materials. More specifically, it is concerned with calibration solutions which are particularly useful in analyzing quantitatively whole-blood samples with dry analysis elements.

BACKGROUND OF THE INVENTION

Dry analysis materials and methods for quantitative analysis of aqueous fluid samples utilizing them are described in U.S. Patents 2,846,808, 3,016,292, 3,036,893, 3,368,872 and 3,552,928.

Dry, multilayer analysis materials composed of a transparent support having thereon at least one reagent layer and a porous layer, in this order, and quantitative analysis methods of aqueous fluid samples using those materials are described, e.g., in U.S. Patents 3,992,158, 3,983,005, 4,042,335, 4,066,403, 4,144,306, 4,132,528, 4,258,001, 4,357,363, 4,381,421 and 4,292,272 and Japanese Patent Application (OPI) NO. 24576.81. (the term "OPI" as used herein means an "unexamined published Japanese patent application"), H.G. Curme et al. and R.W. Spayd et al., Clinical Chemistry, vol. 24, pp. 1,335-1,350 (1978), Bert Walter, Anal. Chem., vol. 55, No. 4, pp. 498-514 (1983) and so on. The feasibility of using as a sample not only diluted serum and blood plasma, but also non-diluted whole blood, is described.

More specifically, examples of clinical tests to determine blood-glucose concentrations within a short time using non-diluted whole blood as a sample using a multilayer-film analytical element are described in Ohkubo et al, Clinical Chemistry, vol 27, pp. 1,287-1,290 (1981).

Such materials typically contain a porous spreading layer that filters out at least a good portion of the solid components of whole blood, allowing the fluid component to pass through to a color-forming reagent layer or layers.

It is required that the porous spreading layer permit a drop of aqueous fluid sample applied thereto first to spread rapidly in a circle in the horizontal direction and then, to penetrate in a vertical direction, supplying the aqueous fluid to the reagent layer located thereunder in an approximately constant volume per unit area. This function is called a spreading function or a metering function. Cotton or polyester fabrics and knits, membrane filter-form nonfibrous isotropic porous materials, porous materials made of bound granules, paper such as filter paper for chemical analysis, e.g., Toyo Roshi No. 2 made by Toyo Roshi Co., Ltd., and so on satisfy these requirements. In particular, employing textile fabrics, knits, or granular constructions of fine granules containing continuous pores described in U.S. Patents 4,258,001, 4,357,363, and 4,381,421 permits the quantitative analysis of whole blood, because those materials possess a spreading function with respect to not only blood plasma and serum but also whole blood containing a solid component.

When the quantitative analysis of a particular analyte in sample solutions, especially whole blood, blood plasma, serum, urin and like samples, is performed in clinics, periodic measurements are generally performed using a solution containing the analyte in a definite amount of order to determine the precision of the analysis system. The solution used is, in general, called a control solution or a standard solution. In addition, solutions containing the analyte in known amounts are used for preparing a calibaration curve. These solutions are generally called calibrators or calibration solutions by those skilled in the art.

Although pooled blood obtained by mixing a number of whole-blood specimens, or pooled sera prepared by mixing serum samples are adopted as a control solution and calibration solutions in some cases, the composition varies with the lot. Further, pooled blood and serum are difficult to preserve. Therefore, calibration solutions are generally simple aqueous solutions containing given amounts of analyte alone, or additionally containing a hydrophilic polymer (e.g., polyvinyl alcohol, polyvinyl pyrrolidone, polyethylene glycol, etc.), plasma protein, or the like. (The aqueous solutions containing plasma protein are, in general, prepared by re-dissolving the lyophilized solid matter in water at the time of use.) When calibration solutions of this kind are applied to the quantative analysis of a particular component in fluid with dry analysis elements, particularly whole-blood analyses with dry analysis elements, the required color-producing reaction does not take place uniformly in the reagent layer(s). Accordingly, the application of such calibration solutions has the disadvantage that within-run reproducibility in color density measurements is poor. Moreover, since there is a difference in response to the analyte content, e.g., in color developability, between such calibration solutions and whole-blood samples, a calibration curve based on such calibration solutions has the defect that it deviates from the true calibration curve of whole-blood samples.

## SUMMARY OF THE INVENTION

An object of the present invention is to improve the precision of the chemical analysis for determining an analyte in whole blood by the use of a dry analysis element, said analyte being solid or liquid at ordinary temperature, i.e., between 15°C to 40°C.

More specifically, an object of the present invention is to provide control or calibration solutions for chemical analysis of whole-blood samples based on the use of dry analysis elements, which, in calibration, have the same response to an analyte content as whole-blood samples, thus providing a calibration curve that matches the true calibration curve of whole-blood samples, and in precision checking of the analysis system, provide excellent reproducibility.

A second object of the present invention is to provide an analysis method which can ensure high accuracy of data by using the above-described control or calibration solutions in making a calibration curve.

The present invention comprises the use of a control or calibration solution in the analytical determination of a particular analyte which is a solid or a liquid at ordinary temperature; using a dry analytical element, characterised in that said solution comprises a water phase, a substance which is the same as or similar to said particular analyte, and a water-insoluble dispersed phase comprising particles from about 0.01 to 10 $\mu$m.

The water-insoluble disperse phase comprises solid or liquid particles which may be present in concentration from about 10 to about 50% by weight.

The invention also comprises a control or calibration solution for the analytical determination of a particular analyte which is a solid or a liquid at ordinary temperature; using a dry analytical element, characterised in that said solution comprises a water phase, a substance which is the same as or similar to said particular analyte, and a water-insoluble phase comprising solid particles of particle size from 0.2 to 2.0 $\mu$m or liquid particles of size from 0.01 to 10 $\mu$m.

U.S. patent 3,977,995 describes a calibrating fluid containing synthetic latex particles for use with automatic instruments for blood cell counting and hemoglobin determination. The calibration fluid is subjected to the instrument to measure optical density of the fluid to the instrument to measure optical density of the fluid or to count the number of the latex particles. In this prior art the particles are used as blood cell count material.

On the other hand, in the present invention, the calibration solution is applied onto the spreading layer of a dry analytical element. The water-insoluble dispersed particles are used in order to make the calibration solution behave for the same as or extremely similar to that of a sample liquid, especially with respect to spreading characteristics at the spreading layer thereby to make the color developability extremely similar to that of the sample liquid, especially whole blood.

## BRIEF DESCRIPTION OF THE DRAWINGS

Fig. 1 and Fig. 2 are diagrams illustrating schematically a whole-blood analysis in which a multilayer-film analytical material is employed as a dry analytical element.

Fig. 3 depicts curves obtained from various kinds of calibration solutions.

## DETAILED DESCRIPTION OF THE INVENTION

The present invention relates to control or calibration solutions containing a definite amount of particular substance being the same as or similar to the substance to be analyzed and a water-insoluble dispersed phase which can be used to check the normality of an analysis system and/or make a calibration curve.

The present invention also relates to an analysis method for determining a particular analyte in whole-blood samples, in which a dry analytical element containing at least one reagent in a water-permeable layer and having a porous spreading layer as the topmost layer is employed as an analyzing means, a calibration curve is determined in advance using calibration solutions containing a water-insoluble dispersed phase, and a definite amount of the particular analyte of which the concentration in whole blood is determined on the basis of the calibration curve.

The present, regulative solutions functioning either as control or calibration solutions, are referred to as a control or calibration solution hereinafter.

The control or calibration solutions of the present invention are now described in greater detail.

A particular substance contained in the control or calibration solution is the same as or similar to the particular analyte to be analyzed and is dissolved or dispersed in the water phase. The amount of the particular substance preferably contained in the water phase is selected from the same range of the amount as that of the analyte in the specimen. Preferable particular substances contained in the control or calibration solution and preferably amounts thereof are shown in the following Table 1. The amount shown in the Table 1 is based on the control or calibration solution.

## Table 1

| Analyte | Particular Substance | *Preferable Addition Amount of Particular Substance | *More Preferable Addition Amount of Particular Substance | Particle diameter |
|---|---|---|---|---|
| Glucose | Glucose | 0 to 2000 mg/dl | 20 to 1000 mg/dl | |
| Urea | Urea | 0 to 1000 mg/dl** | 5 to 200 mg/dl** | |
| Uric Acid | Uric Acid | 0 to 100 mg/dl | 2 to 50 mg/dl | |
| Creatinine | Creatinine | 0 to 100 mg/dl | 0.5 to 50 mg/dl | |
| Bilirubin | Bilirubin | 0 to 100 mg/dl | 0.2 to 50 mg/dl | |
| Hemoglobin | Hemoglobin, Red dyes | 0 to 100 g/dl | 5 to 50 g/dl | |
| Triglyceride | Trioleine | 0 to 2000 mg/dl | 50 to 1000 mg/dl | 0.2 μm or less |
| Triglyceride | Glycerine**** | 0 to 220 mg/dl | 5 to 100 mg/dl | |
| Total cholesterol | Cholesterol | 0 to 2000 mg/dl | 50 to 1000 mg/dl | 0.2 μm or less |
| $NH_4^+$ | $(NH_4)_2SO_4$, $NH_4Cl$ | 0 to 3000 μg/dl** | 20 to 2000 μg/dl** | |
| $Ca^{2+}$ | $CaCl_2$, $Ca(NO_3)_2$ | 0 to 100 mg/dl*** | 5 to 20 mg/dl*** | |
| $Mg^{2+}$ | $MgCl_2$ | 0 to 100 mg/dl | 1 to 10 mg/dl | |
| $Na^+$ | NaCl | 0 to 2000 mg/dl | 20 to 1000 mg/dl | |
| $K^+$ | KCl | 0 to 2000 mg/dl | 2 to 1000 mg/dl | |
| $Cl^-$ | NaCl, KCl, $CaCl_2$ | 0 to 2000 mg/dl | 20 to 1000 mg/dl | |
| $HPO_4^{2-}$ | $K_2HPO_4$ | 0 to 100 mmol | 0.5 to 5 mmol | |
| $SO_4^{2-}$ | $(NH_4)_2SO_4$ | 0 to 100 mmol | 0.1 to 5 mmol | |
| $NO_3^-$ | $Ca(NO_3)_2$, $NaNO_3$ | 0 to 100 mmol | 0.1 to 5 mmol | |

\* Based on the control or calibration solution
\*\* Calculated on the basis of nitrogen
\*\*\* Calculated on the basis of calcium
\*\*\*\*Glycerin is a hydrolysis product of trioleine

Other particular substances used in the present invention also include lipid, neutral fat, various enzymes such as amylase, lactate dehydrogenase.

A dispersion which contains no particular substance may be used in the present invention as a calibrating solution of blank (zero level) calibration solution.

When the water-insoluble dispersed phase is a solid phase, it can be selected from substances including styrene homopolyers; copolymers prepared from styrene and monomers copolymerizable with styrene, acrylate homopolymers, copolymers prepared from acrylates and monomers copolymerizable with acrylates, vinyl acetate homopolymer; copolymers prepared from vinyl acetate and monomers copolymerizable with vinyl acetate, vinyl chloride homopolymers and copolymers prepared from vinyl chloride and monomers copolymerizable with vinyl chloride. Copolymerizing monomers can be chosen from any conventional used comonomers provided that the resulting copolymers are insoluble in water.

The polymers and copolymers described above preferably have from $10^5$ to $10^6$ of molecular weight.

Preferable monomers copolymerizable with the above described monomer include butadiene, vinyl chloride, acrylamide, acrylic acid and, methacrylic acid. The comonomer ratio in the copolymer is preferably from 0 to 90 mol%.

Preferred example of solid particles in the solid phase includes polystyrene having a molecular weight of $10^5$ to $10^6$ and sytrene-acrylic acid copolymer having a comonomer ratio of styrene to acrylic acid of from 50 to 100 mol% and, having a molecular weight of $10^5$ to $10^6$.

When the water-insoluble dispersed phase is a liquid phase, it can be selected from substances including adipates, sebacates, phthalates, trimellitates and phosphates, as disclosed in, Japanese Patent Application (OPI) NO. 122956/81, wherein the alcohol moiety thereof is preferably derived from an alcohol selected from methanol, ethanol, isopropanol, butanol, octanol, decanol, cyclohexanol, phenol, cresol.

Preferred example of liquid particles in the liquid phase includes phthalic acid diesters such as dibutylphthalate, cyclohexylphthalate, di-2-ethylhexylphthalate, decylphthalate, phosphates such as triphenylphosphate, tricresylphosphate, 2-ethylhexyldiphenylphosphate, tricylohexylphosphate, tri-2-ethylhexylphosphate, benzoates such as 2-ethylhexylbenzoate, amides such as N,N-diethyllaurylamide, N-tetradecylpyrrolidone, phenols such as 2,4-di-t-amylphenol, aliphatic esters such as trioctylcitrate, hydrocarbons such as paraffin, halogenated hydrocarbons such as chloroparaffin.

Further, water-insoluble dispersed phase may be an emulsion of natural polymers such as sodium alginate.

The above-described water-insoluble dispersed phase is an emulsion or a suspension of the particle size of which ranges from about 0.01 $\mu$m to about 10 $\mu$m, preferably from about 0.05 $\mu$m to about 6 $\mu$m and most preferably from about 0.2 $\mu$m about 2 $\mu$m. If the phase to be dispersed can be mixed homogeneously with a water phase by a simple stirring operation, the phase may be employed in the present invention, that is, the dispersion or emulsion of the present invention does not need to be stable and it may be used as long as the phase can be dispersed in water as a homogeneous dispersion by simple agitation such as gentle shaking.

Solubility of the water-insoluble particles is preferably less than 0.1 wt% in water.

The water-insoluble particles are contained in an amount of from 1 to 50 wt%, preferably from 10 to 50 wt% in the water-insoluble dispersed phase.s

Other additives which may be contained in the control or calibration solutions include surface active substances, defoaming agents, antiseptics, if desired, provided that they do not adversely affect with the intended analyses. Organic solvents, for example, alcohols, such as methanol, ethanol, benzyl alcohol; and other organic liquid substances can be also added to the control or calibration solutions of the present invention.

Suitable examples of dispersants include a polyethyleneglycol, an alkylphenyl polyethoxy ethanol, e.g., p-nonylphenylpolyethoxy ethanol (containing ethoxy units of 10 to 40).

Suitable examples of the antiseptics include parachlorophenol derivatives and benzothiazole derivatives described in Japanese Patent Application (OPI) Nos. 58765/86 and 89348/86.

The present invention is useful for analyses of particular analytes in fluids by the use of multilayer-film, dry analytical elements described, e.g., in Japanese patent Publication No. 53888/74, Japanese Patent Application (OPI) Nos. 137192/75, 140191/76, 3488/77, 131089/78, 101398/79. 90859/80, 164356/80 and 24576/81. In particular, the invention is useful for analysis of whole blood. Also, it can be utilized in analyzing blood plasma, serum, urine, saliva, Further, it is useful for analyses performed using whole-blood analysis elements as described in Japanese Patent Application (OPI) No. 4959/86, and Japanese Patent Application Nos. 279859/85, 279860/85 and 279861/85.

Examples of analyte include preferably glucose, urine, uric acid, creatinine, hemoglobin, bilirubin, triglyceride. glycerine, cholesterol, $NH_4^+$, $Ca^{2+}$, $Mg^{2+}$, $Na^+$, $K^+$, $Cl^-$, $HPO_4^{2-}$, $SO_4^{2-}$, $NO_3^-$, etc.

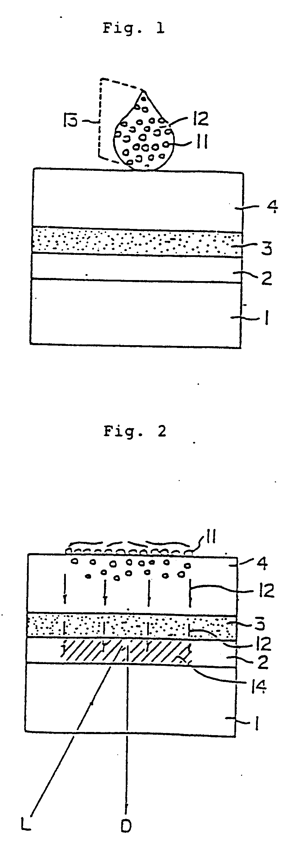

A method of analyzing whole-blood samples using a dry analysis element is illustrated schematically in Fig. 1 and Fig. 2.

The exemplified multilayer-film analytical element is a film analyzer having a multilayer structure provided with a reagent layer 2, light-reflecting layer 3 and a porous spreading layer 4 on one side of a transparent support 1 (of course, plural reagent layers, a barrier layer, a scavenger layer, a buffer layer, a detector layer and so on may be interposed between the support and the spreading layer, if needed). When a blood sample 13 a solid component 11 and a fluid component 12 is spotted on the porous spreading layer 4 of a multilayer analysis film, the whole blood spreads in a circle over the spreading layer, and area of the circle is approximately proportional to the quantity spotted thereon. Then, the fluid component alone passes through the porous spreading layer as the solid component is filtered out, and successively passes through the light-reflecting layer 3, and finally arrives at the reagent layer 2. In principle, a selective color-producing reagent capable of reacting only with a particular analyte in the blood is incorporated in the reagent layer in advance, so color development takes place in proportion to the content of the analyte. The optical density of the color in the color-developer region 14 is measured with a colorimeter from the support side by comparing illuminating light and reflected light, and thereby the content of the analyte in the blood can be determined by colorimetry.

Fig. 1 illustrates the application of a drop of whole blood 13 to the porous spreading layer 4. Fig. 2 illustrates the movement of whole blood after spotting. Specifically, the solid component 11 (blood cells, etc.) is filtered off by the porous spreading layer 4 to remain in the vicinity of the surface of the spreading layer, while the fluid component 12 passed through the light-reflecting layer 3, and reaches the reagent layer 2.

In these figures 1, designates a transparent support, 14 a color-developed region, L the direction of illumination light from a light source, and D the direction of reflected light.

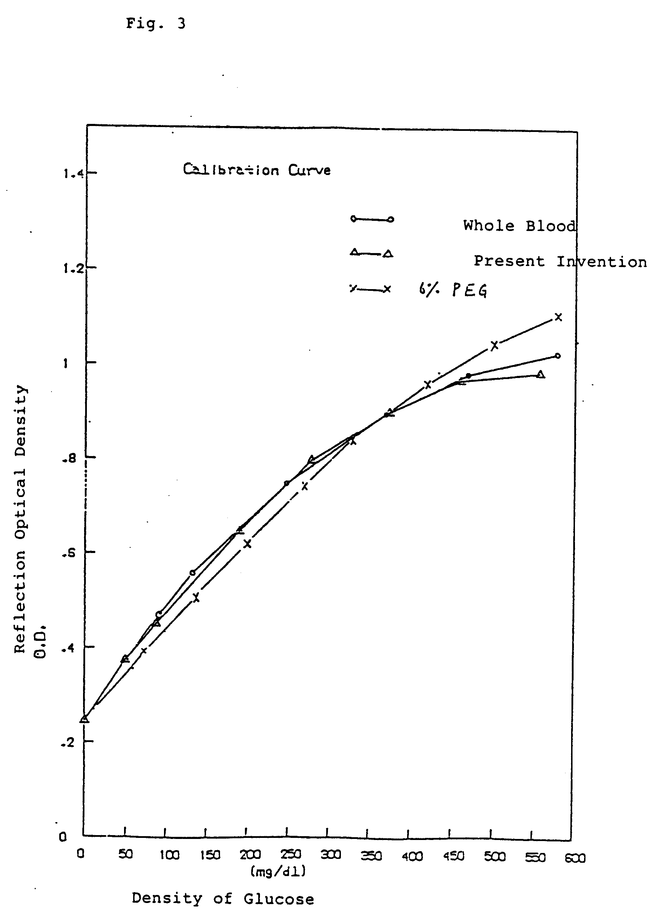

Calibration curves determined from various calibration solutions are shown in Fig. 3.

The present invention is illustrated in greater detail by reference to the following examples.

EXAMPLE 1

In order to determine glucose concentrations in whole-blood samples, dry chemical analysis slides and control solutions were prepared in the following manner.

A coating composition for forming a reagent layer, which contained the following amounts of ingredients, was coated in a dry thickness of 15 $\mu$m on a 180 $\mu$m-thick smooth film of polyethylene terephthalate film having a gelatin substratum, and dried.

| | |
|---|---|
| Gelatin | 20 g |
| Peroxidase | 2,500 IU |
| Glucose oxidase | 1,000 IU |
| 1,7-Dihydroxynaphthalene | 0.5 g |
| 4-aminoantipyrine | 0.5 g |
| Polyoxyethylene nonylphenol (oxyethylene units: about 40)) | 0.2 g |
| Water | 200 ml |

On the reagent layer, a coating composition for forming a light shielding layer, which contained the following amounts of ingredients, was coated in a dry thickness of 7 $\mu$m, and dried.

| | |
|---|---|
| Gelatin | 10 g |
| Titanium dioxide (average particle diameter: 0.3 $\mu$m ) | 100 g |
| Water | 500 ml |

On the light shielding layer, an adhesive composition containing the following amounts of ingredients was coated in a dry thickness of 2 $\mu$m, and dried.

| | |
|---|---|
| Gelatin | 4 g |
| Polyoxyethylene nonylphenol (oxyethylene units: about 40) | 0.1 g |
| Water | 200 ml |

After the adhesive layer was moistened with 30 g/m$^2$ of water, cotton broad cloth (80 yarn counts) was adhere by applying light pressure thereto, and dried.

The thus prepared glucose analysis film was cut into pieces measuring 15 mm by 15 mm in size, and each piece was put on a plastic mount measuring 24 mm by 28 mm in size.

Solutions I and II according to the invention having the following compositions respectively were prepared as a control solution.

| Composition of Solution I: | |
|---|---|
| Acryl-styrene resin (Voncoat PP-1001*) | 500 g |
| Distilled water | 500 g |
| Glucose | 0.650 g |
| Suraofu 72N** | 0.2 g |

| Composition of Solution II: | |
|---|---|
| Acryl-styrene resin (Voncoat PP-1001*) | 500 g |
| Distilled water | 500 g |
| Glucose | 2.100 g |
| Suraofu 72N** | 0.2 g |

\* polystyrene (particle size : 0.2 to 0.6 $\mu$m; solid content (polymer): 50 wt%) produced by Dai-Nippon Ink & Chemicals, Inc.
** antiseptics produced by Takeda Chemical Industries, Ltd.

Of 40 slides for dry analysis of glucose, 20 slides were used for spotting of the foregoing solution I, and the remaining 20 slides were used for spotting of the foregoing solution II. Densities of the developed color were measured with a Fuji DriChem DC-1000 Glucose Analyzer (produced by Fuji Photo Film Co., Ltd.), and within-run reproducibility was examined.

For the purpose of comparison, two control solutions prepared by adding different amounts of glucose to a 7% polyethylene glycol (PEG) solution (molecular weight 20,000), and two fresh whole-blood specimens obtained by adding different amounts of glucose to fresh blood taken with a heparin-coated tube syringe were subjected to the same measurement as described above. Each fluid was spotted on 20 slides in the same manner as above, and reproducibilities thereof were compared with those of the present control solutions.

The results obtained are shown in the following Table 2.

As can be seen from the data in the Table 2, the control solutions of the present invention were excellent in reproducibility (coefficient of variation, abbreviated as CV), compared with the control solutions for comparison, and were equivalent to fresh whole blood in reproducibility.

Table 2

| Within-Run Reproducibility (CV) of Glucose Concentration Determination (mg/dl) | | | | | |
|---|---|---|---|---|---|
| Whole blood | | Invention | | Comparison 7% PEG | |
| 102 | 327 | 112 | 264 | 88 | 292 |
| 102 | 318 | 114 | 261 | 86 | 292 |
| 106 | 319 | 113 | 277 | 89 | 294 |
| 102 | 314 | 113 | 271 | 90 | 295 |
| 105 | 325 | 115 | 269 | 89 | 294 |
| 105 | 316 | 113 | 273 | 90 | 288 |
| 104 | 316 | 114 | 272 | 87 | 288 |
| 105 | 319 | 115 | 264 | 89 | 283 |
| 107 | 320 | 114 | 267 | 89 | 283 |
| 105 | 316 | 111 | 264 | 90 | 279 |
| 104 | 312 | 111 | 270 | 88 | 289 |
| 107 | 318 | 113 | 272 | 93 | 292 |
| 106 | 314 | 112 | 266 | 91 | 297 |
| 102 | 313 | 113 | 266 | 90 | 292 |
| 105 | 312 | 113 | 270 | 86 | 292 |
| 105 | 314 | 115 | 264 | 87 | 283 |
| 102 | 319 | 116 | 261 | 86 | 262 |
| 104 | 316 | 115 | 273 | 86 | 288 |
| 104 | 327 | 117 | 272 | 89 | 292 |
| 105 | 348 | 114 | 269 | 88 | 289 |
| $\bar{X}$  104 | 318 | 114 | 268 | 89 | 288 |
| SD  1.63 | 4.57 | 1.53 | 4.23 | 1.88 | 7.75 |
| CV  1.56 | 1.44 | 1.34 | 1.58 | 2.12 | 2.69 |
| $\bar{X}$ designates an average value | | | | | |

As is apparent from the results, comparative solution containing 7% of polyethylene glycol, which does not compose a dispersed phase provides a large C. V. value of over 2% or less. On the other hand, the calibration solution of the present invention porvides a C.V. value of 2% or less, which value is almost the same as that of whole blood, and, thus, reproducibility is excellent.

EXAMPLE 2

For the purpose of determination of glucose concentrations, dry chemical analysis slides and calibration solutions were prepared in the same manner as in Example 1.

Solutions III, IV and V having the following compositions respectively were prepared as primary glucose calibration solutions.

| Composition of Solution III: | |
|---|---|
| Distilled water | 360 g |
| Glucose | 0.500 g |
| Cebian A46774* | 640 g |
| Suraofu 72N** | 0.2 g |

*styrene-ethylacrylate copolymer (molar ratio 1:4; particle size average 0.3 $\mu$m; polymer content 30wt%) produced by Daicel Ltd.

**antiseptics produced by Takeda Chemical Industries Ltd.

| Composition of Solution IV: | |
|---|---|
| Distilled water | 360 g |
| Glucose | 2.000 g |
| Distilled water | 360 g |

| Composition of Solution III: | |
|---|---|
| Cebian A46774* | 640 g |
| Suraofu 72N** | 0.2 g |

*styrene-ethylacrylate copolymer (molar radio 1:4; particle size:average 0.3 $\mu$m; polymer content 30wt%) produced by Daicel Ltd.
**antiseptics produced by Takeda Chemical Industries Ltd.

| Composition of Solution V: | |
|---|---|
| Distilled water | 360 g |
| Glucose | 5.000 9 |
| Cebian A46774* | 640 g |
| Suraofu 72N** | 0.2 g |

*styrene-ethylacrylate copolymer (molar ratio 1:4; particle size:average 0.3 $\mu$m; polymer content 30wt%) produced by Daicel Ltd.
**antiseptics produced by Takeda Chemical Industries Ltd.

The following three kinds of calibration solutions (a), (b) and (c) were prepared.

(a) Eight invention calibration solutions prepared by mixing the foregoing three primary solutions so that their respective glucose concentrations were within the range of from 0 to 600 mg/dl.

(b) Eight specimens of wholeblood, which were prepared so as to have their respective glucose concentrations within the range of from 0 to 600 mg/dl.

(c) Eight comparison solutions, which were prepared by mixing three primary solutions containing 6 wt% of polyethylene glycol (molecular weight 20,000) and 0.500, 2.000 and 5.000 g/l of glucose, respectively.

Each of eight comparison calibration solutions of the present invention was spotted on three separate glucose-analysis slides described above. Also, eight whole-blood specimens for calibration and eight calibration solutions were spotted similarly. Densities of developed colors in these slides were measured with a Fuji DriChem DC-100 Glucose Analyzer (made by Fuji Photo Film Co., Ltd.). The dDensity data of colors developed in the three slides under the same condition were averaged. The results obtained are shown in Fig. 3.

As can be seen from the graph of Fig. 3, the calibration curve obtained from the calibration solutions of the present invention was closer to that of whole blood than that obtained from the comparative calibration solutions.

## Claims

1. The use of a control or calibration solution in the analytical determination of a particular analyte which is a solid or a liquid at ordinary temperature, using a dry analytical element, characterised in that said solution comprises a water phase, a substance which is the same as or similar to said particular analyte, and a water-insoluble dispersed phase comprising particles from about 0.01 to 10 $\mu$m.

2. The use of a control or calibration solution as claimed in claim 1, wherein said water-insoluble dispersed phase comprises solid particles.

EP 0 266 216 B1

**3.** The use of a control or calibration solution as claimed in claim 1, wherein said water-insoluble dispersed phase comprises liquid particles.

**4.** The use of a control or calibration solution as claimed in claim 1, 2 or 3 wherein said water-insoluble dispersed phase is present in a concentration of about 10 to 50% by weight.

**5.** The use of a control or calibration solution as claimed in any of claims 1 to 4, wherein said analyte comprises a component of whole blood.

**6.** The use of a control or calibration solution as claimed in any of the preceding claims, wherein said particular substance is selected from the group consisting of glucose, urea, uric acid, creatinine, bilirubin, haemoglobin, trioleine, glycerine, cholesterol, $(NH_4)_2SO_4$, $NH_4Cl$, $CaCl_2$, $Ca(NO_3)_2$, $MgCl_2$, NaCl, KCl, $K_2HPO_4$, $Ca(NO_3)_2$, and $NaNO_3$.

**7.** The use of a control or calibration solution as claimed in any of the preceding claims, wherein the water-insoluble dispersed phase comprises solid particles selected from the group consisting of styrene homopolymers, copolymers prepared from styrene and monomers copolymerizable with styrene, acrylate homopolymers, copolymers prepared from acrylates and monomers copolymerizable with acrylates, vinyl acetate homopolymers, copolymers prepared from vinyl acetate and monomers copolymerizable with vinyl acetate, vinyl chloride homopolymers and copolymers prepared from vinyl chloride and monomers copolymerizable with vinyl chlorides.

**8.** The use of a control or calibration solution as claimed in any of claims 1 to 5, wherein the water-insoluble dispersed phase comprises liquid particles selected from the group consisting of adipates, sebacates, phthalates, trimellitates and phosphates.

**9.** The use of a control or calibration solution as claimed in any of the preceding claims wherein the dry analytical element contains at least one reagent in a water-permeable layer and has a porous spreading layer as the topmost layer in the element.

**10.** The use of a control or calibration solution according to any of the preceding claims in which the dispersed phase is of particle size from 0.05 to 6 $\mu$m.

**11.** A control or calibration solution for the analytical determination of a particular analyte which is a solid or a liquid at ordinary temperature, using a dry analytical element, characterised in that said solution comprises a water phase, a substance which is the same as or similar to said particular analyte, and a water-insoluble dispersed phase comprising solid particles of particle size from 0.2 to 2.0 $\mu$m.

**12.** A control or calibration solution for the analytical determination of a particular analyte which is a solid or a liquid at ordinary temperature; using a dry analytical element, characterised in that said solution comprises a water phase, a substance which is the same as or similar to said particular analyte, and a water-insoluble dispersed phase comprising liquid particles of particle size from 0.01 to 10 $\mu$m.

**Patentansprüche**

**1.** Die Verwendung einer Kontroll- oder Eichlösung bei der analytischen Bestimmung eines besonderen Analyten, der bei üblicher Temperatur fest oder flüssig ist, unter Verwendung eines trockenen analytischen Elements, dadurch **gekennzeichnet,** daß die Lösung eine Wasserphase, eine Substanz, die gleich oder ähnlich ist wie der genannte besondere Analyt und eine wasserunlösliche dispergierte Phase, die Teilchen von etwa 0,01 bis 10 $\mu$m enthält, umfaßt.

**2.** Die Verwendung einer Kontroll- oder Eichlösung nach Anspruch 1, dadurch **gekennzeichnet,** daß die wasserunlösliche dispergierte Phase feste Teilchen enthält.

**3.** Die Verwendung einer Kontroll- oder Eichlösung nach Anspruch 1, dadurch **gekennzeichnet,** daß die wasserunlösliche dispergierte Phase flüssige Teilchen enthält.

11

**4.** Die Verwendung einer Kontroll- oder Eichlösung nach Anspruch 1, 2 oder 3, dadurch **gekennzeichnet,** daß die wasserunlösliche dispergierte Phase in einer Konzentration von etwa 10 bis 50 Gew.-% vorhanden ist.

**5.** Die Verwendung einer Kontroll- oder Eichlösung nach einem der Ansprüche 1 bis 4, dadurch **gekennzeichnet,** daß der Analyt eine Komponente von Gesamtblut umfaßt.

**6.** Die Verwendung einer Kontroll- oder Eichlösung nach einem der vorhergehenden Ansprüche, dadurch **gekennzeichnet,** daß die besondere Substanz ausgewählt wird aus der Gruppe, die besteht aus Glukose, Harnstoff, Urinsäure, Creatinin, Bilirubin, Hämoglobin, Triolein, Glycerin, Cholesterin, $(NH_4)_2SO_4$, $NH_4Cl$, $CaCl_2$, $Ca(NO_3)_2$, $MgCl_3$, $NaCl$, $KCl$, $K_2HPO_4$, $Ca(NO_3)_2$ und $NaNO_3$.

**7.** Die Verwendung einer Kontroll- oder Eichlösung nach einem der vorhergehenden Ansprüche, dadurch **gekennzeichnet,** daß die wasserunlösliche dispergierte Phase feste Teilchen ausgewählt aus der Gruppe, die besteht aus Styrolhomopolymerisaten, Copolymerisaten hergestellt aus Styrol und Monomeren, die mit Styrol copolymerisierbar sind, Acrylathomopolymerisaten, Copolymerisaten, die aus Acrylaten und Monomeren, die mit Acrylaten copolymerisierbar sind, hergestellt worden sind, Vinylacetathomopolymerisaten, Copolymerisaten, die aus Vinylacetat und Monomeren, die mit Vinylacetat copolymerisierbar sind, hergestellt worden sind, Vinylchloridhomopolymerisaten und Copolymerisaten, die aus Vinylchlorid und Monomeren, die mit Vinylchlorid copolymerisierbar sind, hergestellt worden sind, umfaßt.

**8.** Die Verwendung einer Kontroll- oder Eichlösung nach einem der Ansprüche 1 bis 5, dadurch **gekennzeichnet,** daß die wasserunlösliche dispergierte Phase flüssige Teilchen ausgewählt aus der Gruppe, die besteht aus Adipaten, Sebacaten, Phthalaten, Trimellitaten und Phosphaten, umfaßt.

**9.** Die Verwendung einer Kontroll- oder Eichlösung nach irgendeinem der vorhergehenden Ansprüche, dadurch **gekennzeichnet,** daß das trockene analytische Element mindestens ein Reagenz in einer wasserpermeablen Schicht und eine porösen Ausbreitungsschicht als oberste Schicht in dem Element enthält.

**10.** Die Verwendung einer Kontroll- oder Eichlösung nach irgendeinem der vorhergehenden Ansprüche, dadurch **gekennzeichnet,** daß die dispergierte Phase eine Teilchengröße von 0,05 bis 6 $\mu$m besitzt.

**11.** Kontroll- oder Eichlösung für die analytische Bestimmung eines besonderen Analyten, der bei üblicher Temperatur fest oder flüssig ist, unter Verwendung eines trockenen analytischen Elements, dadurch **gekennzeichnet,** daß die Lösung eine Wasserphase, eine Substanz, die gleich oder ähnlich ist wie der besondere Analyt und eine wasserunlösliche dispergierte Phase, die feste Teilchen mit einer Teilchengröße von 0,2 bis 2,0 $\mu$m enthält, umfaßt.

**12.** Kontroll- oder Eichlösung für die analytische Bestimmung eines besonderen Analyten, der bei üblicher Temperatur fest oder flüssig ist, unter Verwendung eines trockenen analytischen Elements, dadurch **gekennzeichnet,** daß die Lösung eine Wasserphase, eine Substanz, die gleich oder ähnlich ist wie der besondere Analyt, und eine wasserunlösliche dispergierte Phase, die flüssige Teilchen einer Teilchengröße von 0,01 bis 10 $\mu$m enthält, umfaßt.

**Revendications**

**1.** Utilisation d'une solution de contrôle ou d'étalonnage pour la détermination analytique d'un produit d'analyse particulier consistant en un solide ou un liquide à la température ordinaire, au moyen d'un élément d'analyse sec, caractérisée en ce que la solution comprend une phase d'eau, une substance identique ou analogue au produit d'analyse particulier, et une phase dispersée insoluble dans l'eau comprenant des particules d'environ 0,01 à 10 x $10^{-6}$ m.

**2.** Utilisation d'une solution de contrôle ou d'étalonnage selon la revendication 1, caractérisée en ce que la phase dispersée insoluble dans l'eau comprend des particules solides.

EP 0 266 216 B1

**3.** Utilisation d'une solution de contrôle ou d'étalonnage selon la revendication 1, caractérisée en ce que la phase dispersée insoluble dans l'eau comprend des particules liquides.

**4.** Utilisation d'une solution de contrôle ou d'étalonnage selon l'une quelconque des revendications 1 à 3, caractérisée en ce que la phase dispersée insoluble dans l'eau est présente à une concentration d'environ 10 à 50 % en poids.

**5.** Utilisation d'une solution de contrôle ou d'étalonnage selon l'une quelconque des revendications 1 à 4, caractérisée en ce que le produit d'analyse comprend un élément de sang complet.

**6.** Utilisation d'une solution de contrôle ou d'étalonnage selon l'une quelconque des revendications précédentes, caractérisée en ce que la substance particulière est choisie dans le groupe comprenant le glucose, l'urée, l'acide urique, la créatinine, la bilirubine , l'hémoglobine, la trioléine, la glycérine, le cholestérol, $(NH_4)_2SO_4$, $NH_4Cl$, $CaCl_2$, $Ca(NO_3)_2$, $MgCl_2$, $NaCl$, $KCl$, $K_2HPO_4$, $Ca(NO_3)_2$ et $NaNO_3$.

**7.** Utilisation d'une solution de contrôle ou d'étalonnage selon l'une quelconque des revendication précédentes, caractérisée en ce que la phase dispersée insoluble dans l'eau comprend des particules solides choisies dans le groupe comprenant des homopolymères de styrène, des copolymères préparés à partir de styrène et de monomères copolymérisables avec le styrène, des homopolymères d'acrylates, des copolymères préparés à partir d'acrylates et de monomères copolymérisables avec les acrylates, des homopolymères d'acétate de vinyle, des copolymères préparés à partir d'acétate de vinyle et de monomères copolymérisables avec l'acétate de vinyle, des homopolymères et des copolymères chlorure de vinyle préparés à partir de chlorure de vinyle et de monomères copolymérisables avec le chlorure de vinyle.

**8.** Utilisation d'une solution de contrôle ou d'étalonnage selon l'une quelconque des revendications 1 à 5, caractérisée en ce que la phase dispersée insoluble dans l'eau comprend des particules liquides choisies dans le groupe comprenant des adipates, des sébacates, des phthalates, des trimellitates et des phosphates.

**9.** Utilisation d'une solution de contrôle ou d'étalonnage selon l'une quelconque des revendications précédentes, caractérisée en ce que l'élément d'analyse sec contient au moins un réactif dans une couche perméable à l'eau et comporte une couche d'étalement poreuse constituant la couche supérieure de l'élément.

**10.** Utilisation d'une solution de contrôle ou d'étalonnage selon l'une quelconque des revendications précédentes, caractérisée en ce que la phase dispersée est constituée de particules dont la taille est comprise entre 0,05 et $6 \times 10^{-6}$ m.

**11.** Solution de contrôle ou d'étalonnage pour la détermination analytique d'un produit d'analyse particulier constitué par un solide ou un liquide à la température ordinaire, en utilisant un élément d'analyse sec, solution caractérisée en ce qu'elle comprend une phase d'eau, une substance identique ou analogue au produit d'analyse particulier, et une phase dispersée insoluble dans l'eau comprenant des particules solides dont la taille est comprise entre 0,2 et $2,0 \times 10^{-6}$ m.

**12.** Solution de contrôle ou d'étalonnage pour la détermination analytique d'un produit d'analyse particulier constitué par un solide ou un liquide à la température ordinaire, en utilisant un élément d'analyse sec, solution caractérisée en ce qu'elle comprend une phase d'eau, une substance identique ou analogue au produit d'analyse particulier, et une phase dispersée insoluble dans l'eau comprenant des particules liquides dont la taille est comprise entre 0,01 et $10 \times 10^{-6}$ m.

13

Fig. 1

Fig. 2

Fig. 3

Calibration Curve

○——○ Whole Blood
△——△ Present Invention
×——× 6% PEG

Reflection Optical Density
O.D.

(mg/dl)

Density of Glucose